# EUROPEAN PATENT APPLICATION

(11) **EP 1 645 983 A1**
(43) Date of publication of application: **12.04.2006**
(21) Application number: 05256220.4
(22) Date of filing: 05.10.2005
(51) Int. Cl.: G06F 19/00

(54) **Medical data acquisition system**

(30) Priority: 08.10.2004 US 617232 P
(71) Applicant: Draeger Medical Systems, Inc., Danvers, MA 01923 (US)
(72) Inventor: Carter, Linda, Luray Virginia 22835 (US); Shaffer, Judith, Easton, CT 06612 (US); Manetta, Amy, North Billerica MA 01862 (US); Rutledge, Jolyn, Amesbury MA 01913 (US); Penny, Mark, Newburyport MA 01950 (US); Xu, Shuyi, Reading MA 01867 (US)
(74) Representative: Vinsome, Rex Martin

(57) **Abstract**

A system for acquiring and providing data relating to an entity to a user provides for specification of a user defined time "keeper" or anchor for navigation through an electronic data storage system such that the data displayed from screen to screen is kept in the same time context without requiring user scrolling or multiple mouse clicks. Such a system includes at least one repository (102) of entity related data and information indicating an index associated with the entity related data. A source (130) of configuration data identifies a particular index value for the entity. An acquisition processor (104) accesses the repository to acquire entity related data associated with the particular index value in response to a user command to access entity related data.

## Description

### FIELD OF THE INVENTION

The present invention relates to a data acquisition system and in particular to a medical data acquisition system including a user interface.

### BACKGROUND OF THE INVENTION

Data acquisition and storage systems can store large amounts of data related to an entity; more than can reasonably be displayed on one display screen. In many cases an individual datum is related to other data by its location within a series of data gathering points. For example, in meteorology, atmospheric data is gathered at geographic locations on the earth's surface separated by a predetermined distance and/or at different locations within the atmosphere at altitudes separated by a predetermined height. In mechanical engineering, mechanical parameters are measured at locations on a structure separated by a predetermined distance. For example, strain gages measure strain at predetermined locations along a beam under test. Similarly, many data gathering applications gather data at predetermined time intervals. More specifically, physiological data (e.g. (a) blood pressure parameters, (b) ventilation parameters, (c) vital sign parameters, (d) blood oxygen concentration representative parameters and (e) infusion pump parameters associated with fluid delivery), is often taken at predetermined time intervals from a patient.

When data is requested, most data access systems display data at or around a default data gathering point, such as a predetermined time. For example, in medical systems, the most often desired, and therefore the default time location, is the latest data. Thus, when a clinician requests patient physiological data of a particular type, such as blood pressure parameters, the latest data is automatically displayed by default. Should the clinician require data from a different time, the clinician controls the data access system to navigate to, acquire and display the desired data, usually by some combination of keystrokes and/or mouse operations.

Patient medical data (e.g. chart data) for a patient's hospital length of stay may consist of a large amount of data, especially if the patient has been admitted for a long period of time. Such data includes, for example, graphical information such as the 12 waveforms in a 12 lead EKG and/or respiration loops, and textual information such as blood pressure parameters, ventilation parameters, vital signs, blood oxygen levels and so forth. As described above, often there is too much data to be displayed on one display screen at any one time. Thus, a clinician accesses one type of patient data to be displayed, then a different type, and so forth. If the clinician is interested in a different time than the default latest data, then when each screen of data is displayed, the clinician navigates to the desired time by performing the appropriate keypress and mouse operations, described above, to locate and display the requested data at the desired time location. Such repeated navigation is time inefficient and cumbersome. A system according to invention principles addresses these deficiencies and related problems.

### BRIEF SUMMARY OF THE INVENTION

According to an aspect of the present invention, there is provided a system for acquiring and providing data relating to an entity to a user, comprising:
at least one repository of entity related data together with information representing an index associated with acquisition of said entity related data;
a source of configuration data identifying a particular value for said index; and
an acquisition processor for accessing said at least one repository to acquire entity related data associated with said particular index value in response to a user command to access entity related data.

According to another aspect of the present invention, there is provided a system for acquiring and providing particular patient medical record data to a user, comprising;
at least one repository of patient medical record data together with information representing a time associated with acquisition of said medical record data;
a source of configuration data identifying a particular time for a particular patient; and
an acquisition processor for accessing said at least one repository to acquire patient medical record data associated with said particular time in response to user command to access medical record data of said particular patient.

According to a further aspect of the present invention, there is provided a system supporting navigability and interoperability between different executable applications, comprising:
at least one repository including a plurality of different types of data together with information representing a time associated with data within a particular type, said plurality of different types of data being associated with a plurality of different entities;
first and second different executable applications having access to configuration data identifying a particular time associated with a particular type of data and a particular entity; and
said first and second different executable applications access said at least one repository to acquire a particular type of data associated with said particular time in response to user command to access a particular type of data associated with a particular entity.

According to a further aspect of the present invention, there is provided a method of acquiring and providing entity related data to a user, comprising the activities of:
storing, in at least one repository, entity related data together with information representing an index value associated with acquisition of said entity related data;
receiving user entered configuration data identifying a particular index value; and
accessing said at least one repository to acquire entity related data associated with said particular index value in response to user command to access entity related data.

According to a further aspect of the present invention, there is provided a method for acquiring and providing particular patient medical record data to a user, comprising the activities of:
storing, in at least one repository, patient medical record data together with information representing a time associated with acquisition of said medical record data;
receiving user entered configuration data identifying a particular time for a particular patient; and
accessing said at least one repository to acquire patient medical record data associated with said particular time in response to user command to access medical record data of said particular patient.

A system for acquiring and providing data relating to an entity to a user according to invention principles provides for specification of a user defined time "keeper" or anchor for navigation through an electronic data storage system such that the data displayed from screen to screen is kept in the same time context without requiring user scrolling or multiple mouse clicks. In accordance with principles of the present invention, such a system includes at least one repository of entity related data and information indicating an index associated with the entity related data. A source of configuration data identifies a particular index value for the entity. An acquisition processor accesses the repository to acquire entity related data associated with the particular index value in response to a user command to access entity related data.

### BRIEF DESCRIPTION OF THE DRAWING

In the drawings:
Fig. 1 is a block diagram of a data acquisition system according to principles of the present invention;
Fig. 2 is an image window of a GUI for allowing a user to set a particular index value according to invention principles;
Fig. 3 is an image window of a GUI for displaying a report including entity related data retrieved according to the present invention;
Fig. 4 is an image window of a GUI for disabling use of a particular index value according to invention principles;
Fig. 5 is a data flow diagram of executable applications, containing executable procedures, according to principles of the present invention; and
Fig. 6 is a block diagram of a processing system on which the system illustrated in Fig. 1 may be implemented according to invention principles.

### DETAILED DESCRIPTION OF THE INVENTION

A processor, as used herein, operates under the control of an executable application to (a) receive information from an input information device, (b) process the information by manipulating, analyzing, modifying, converting and/or transmitting the information, and/or (c) route the information to an output information device. A processor may use, or comprise the capabilities of, a controller or microprocessor, for example. The processor may operate with a display processor. A display processor is a known element for generating signals representing display images or portions thereof. A processor and a display processor comprises any combination of, hardware, firmware, and/or software.

An executable application, as used herein, comprises code or machine readable instructions for conditioning the processor to implement predetermined functions, such as those of an operating system, data acquisition system or other information processing system, for example, in response user command or input. An executable procedure is a segment of code or machine readable instruction, sub-routine, or other distinct section of code or portion of an executable application for performing one or more particular processes. These processes may include receiving input data and/or parameters, performing operations on received input data and/or performing functions in response to received input parameters, and providing resulting output data and/or parameters. A calling procedure is a procedure for enabling execution of another procedure, e.g. a called procedure, subprocedure or subroutine, in response to a received command or instruction. An object as used herein comprises a grouping of data, executable instructions or a combination of both or an executable procedure.

A user interface comprises one or more display images, generated by the display processor under the control of the processor, enabling user interaction with a processor or other device.

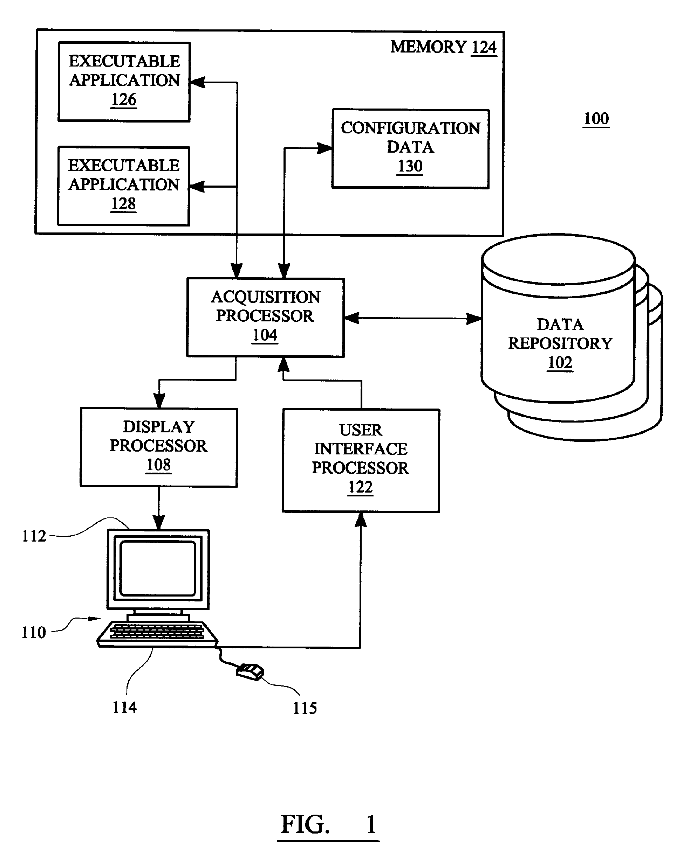

Fig. 1 is a block diagram of a data acquisition system 100 according to principles of the present invention. In Fig. 1, a data repository 102 contains data related to an entity, such as the atmosphere, a structure or a patient. The data repository 102 also contains information representing an index associated with acquisition of the entity related data. The index information represents proximity of data received from different sampling points. An exemplary arrangement of entity related data within the data repository 102 is illustrated in Table 1 (below). This arrangement is schematic only and not meant to represent physical arrangement of data within the data repository 102.

In Table 1, each row represents one entity related datum or one set of data gathered at one sampling point and respective columns represent the entity related data value(s) and the index value associated with that data value(s). More specifically, in the illustrated embodiment, a datum value d-1 is associated with an index value n-1; a datum value d is associated with an index value n and a datum value d+1 is associated with an index value n+1, and so forth.

**Table 1**

| **Entity related data** | |
|---|---|
| **Index** | **Data** |
| ... | ... |
| ... | ... |
| n | d |
| n+1 | d+1 |
| ... | ... |

One skilled in the art understands that the index value may represent a physical point, such as the coordinates of a geographical location, the distance of a data sampling point on a structure from a reference point, altitude of a data sampling point above mean sea level, and so forth. The index value may also represent a time value, which may be a sample number (e.g. sample 1, 2, 3, ... n-1, n, n+1 ...), an actual time, or any similar way of identifying neighboring data samples. A time index may be represented by the displacement from a predetermined time (e.g. the number of seconds since 0000 GMT January 1, 1970) or by an actual time and date (e.g. 1:25 and 37.4 seconds p.m. GMT, on September 2, 2005). The former may be used in cases where a time difference between data samples is important. The latter may be used in cases where time of day is important, such as physiological data in medical applications where the time of day may be important in interpreting the data.

Table 2 (below) illustrates a portion of patient data stored in the data repository 102. In Table 2, data representing blood pressure information is illustrated. The blood pressure data consists of a systolic pressure, a diastolic pressure and a heart rate. One skilled in the art understands that other records (not shown) may contain other physiological data (e.g. ventilation parameters, vital sign parameters, blood oxygen concentration representative parameters, infusion pump parameters, etc.) from the patient. In Table 2, the index consists of a date and a time-of-day. The combination of the index and medical data fields represents a particular sample or reading of the blood pressure data.

**Table 2**

| **Patient medical record data** | | | | |
|---|---|---|---|---|
| **Index** | | Medical data | | |
| **Date** | **Time** | **Systolic** | **Diastolic** | **HR** |
| ... | ... | ... | ... | ... |
| 9/2/05 | 13:00 | 162 | 94 | 92 |
| 9/2/05 | 13:32 | 158 | 91 | 85 |
| 9/2/05 | 14:01 | 147 | 87 | 80 |
| ... | ... | ... | ... | ... |

The data repository 102 may also contain data related to a plurality of entities. For example, the data repository 102 may contain strain data related to a plurality of beams in a structure, or physiological data related to a plurality of patients. In this case, data identifying the particular entity (e.g. patient) is also stored in the data repository 102. Table 3 (below) illustrates a portion of data stored in the data repository 102 for a plurality of patients. As in Table 2 (above), Table 3 illustrates blood pressure information, though entries containing other physiological data may also be stored. A "Patient" column contains data identifying a patient. In Table 3 that data is illustrated as a patient name. One skilled in the art understands that this data may also be a patient medical record number, or other such identification data. Also as in Table 2 (above), Table 3 includes an index consisting of a date and a time of day.

**Table 3**

| **Multiple patient medical record data** | | | | | |
|---|---|---|---|---|---|
| **Patient** | **Index** | | **Medical data** | | |
| | **Date** | **Time** | **Systolic** | **Diastolic** | **HR** |
| ... | ... | ... | ... | ... | ... |
| Jones | 9/5/05 | 11:54 | 124 | 82 | 55 |
| Jones | 9/5/05 | 15:02 | 126 | 84 | 62 |
| Jones | 9/5/05 | 17:58 | 132 | 88 | 72 |
| ... | ... | ... | ... | ... | ... |
| Smith | 9/2/05 | 13:00 | 162 | 94 | 92 |
| Smith | 9/2/05 | 13:32 | 158 | 91 | 85 |
| Smith | 9/2/05 | 14:01 | 147 | 87 | 80 |
| ... | ... | ... | ... | ... | ... |

One skilled in the art understands that the data repository 102 illustrated in Fig. 1 may represent more than one data repository. These data repositories 102 may store respectively different types of data (e.g. one may store ECG data, another store blood pressure data, and so forth); or may store related data (ventilation data, vital signs data, and so forth. The data may instead be replicated and/or distributed among the different data repositories to increase security and reliability; or data may be distributed among the data repositories 102 according to some combination of these or according to some other scheme for allocating data to the one or more data repositories represented by 102. One skilled in the art further understands that the entity related data may be stored in the data repository 102 in a database, either flat file, relational, hierarchical or any other database structure; or in any other manner providing for storage and retrieval of such data.

A bidirectional terminal of an acquisition processor 104 is coupled to a corresponding terminal of the data repository 102. An output terminal of the acquisition processor 104 is coupled to an input terminal of a display processor 108. An output terminal of the display processor 108 is coupled to a monitor 112 of a user interface device 110. User input devices, specifically a keyboard 114 and a mouse 115, are coupled to an input terminal of a user interface processor 122. An output terminal of the user interface processor 122 is coupled to an input terminal of the acquisition processor 104. The acquisition processor 104 is also bidirectionally coupled to a memory 124. The memory 124 contains a first executable application 126, a second executable application 128, and a storage area 130 containing configuration data which are bidirectionally coupled to the acquisition processor 104.

The operation of the acquisition processor 104 is controlled by the machine readable instructions making up the first and second executable applications 126 and 128. In general, these instructions condition the acquisition processor 104 to display an image on the monitor 112 via the display processor 108, and to receive user input from the input devices, keyboard 114 and mouse 115, via the user interface processor 122. The combination of the displayed image on the monitor 112 and the input devices 114 and 115 form a graphical user interface (GUI). Data, parameters and commands may be displayed for and received from the user via the GUI.

The user commands may invoke execution of machine readable instructions making up an executable procedure to acquire entity related data from the data repository 102. As described above, entity related data is typically retrieved from the data repository 102 around a default index value. For example, in medical data storage and acquisition systems, the most recent data is retrieved. This data is then processed by the acquisition processor 104, under the control of the executable procedure, to condition the display processor 108 to generate a signal representing an image displaying the retrieved physiological data. This image representative signal is supplied to the monitor 112 which displays the image.

There is typically more than one type of entity related data. For example, in structural analysis data acquisition systems data representing strain, temperature, elongation, etc. is stored in the data repository 102. Similarly, in medical physiological data acquisition systems data representing different physiological parameters, e.g. (a) blood pressure parameters, (b) ventilation parameters, (c) vital sign parameters, (d) blood oxygen concentration representative parameters, (e) infusion pump parameters associated with fluid delivery, etc., is stored in the data repository 102. The user, interacting with the GUI, selects a type of entity related data. Data representing the selected entity related data type is retrieved from the data repository 102 and displayed on the monitor 112. If the user wants to inspect a different type of entity related data, the new desired data type is selected, and the desired data is retrieved from the data repository 102 and displayed on the monitor 112.

As described above, the user may sometimes want to inspect entity related data at and/or around a different index value than the default. For example, in medical data acquisition systems, a user may wish to examine data at a time earlier than the most recent. To do this in prior systems, the user interacts with the GUI displayed in the monitor 112 using the user input devices, keyboard 114 and/or the mouse 115 to navigate to data associated with a different index value (e.g. time). The newly desired entity related data is retrieved from the data repository 102 and displayed on the monitor 112. As before, if a different type of entity related data is desired, the user selects the new data type, the most recent desired data is retrieved from the data repository 102 and displayed on the monitor 112. The user then navigates to the desired time using the GUI displayed on the monitor 112 and the user input devices keyboard 114 and/or mouse 115.

In accordance with principles of the present invention, a user may, however, specify a particular index value, which will be used as the default index value until it is changed by the user. This particular index value may, for example, be a specific location on a structure where a structural failure occurred; or may be a specific time at which a physiological event of interest occurred. Using the GUI, the user may specify a new particular index value, may change the particular index value, or may reset the index value to the normal or typical default. The particular index value specified by the user using the GUI is supplied to the acquisition processor 104 via the user interface processor 122. The acquisition processor 104 stores the particular index value in the configuration data 130 in the memory 124.

As described above, the data repository 102 may store data related to more than one entity. Table 4 (below) illustrates a table structure which may be used to store configuration data 130 in the memory 124 for such a case. In the illustrated embodiment, Table 4 (below) is related to a medical physiological data acquisition system. Each row contains a particular index value for an associated entity. More specifically, in the illustrated embodiment, the entity is a patient and the index value is a date and time. A first column includes identification information for the patient. In Table 4 (below), this information is a textual representation of the patient name, but it may be a patient identifier or medical record number (not shown). A second column contains a date and a third column includes a time-of-day. The date and time-of-day, in combination, form the particular index value. In Table 4 (below), the date and time-of-day for the patient "Smith" is "<<null>>". This indicates that no particular index value is specified and the normal default is to be used (e.g. most recent data).

**Table 4**

| **Entity** | **Particular index value** | |
|---|---|---|
| | **Date** | **Time** |
| ... | ... | ... |
| Jones (ID: 23) | 29-June-2004 | 00:00 |
| ... | ... | ... |
| Smith (ID:58) | «null» | «null» |
| ... | ... | ... |

One skilled in the art understands that the configuration data 130 may be stored in the memory 124 in a database structure, such as a flat file, relational, hierarchical or any other database structure; or may be stored in any other fashion allowing storing and retrieving of the desired data. One skilled in the art also understands that the memory 124 is volatile memory, meaning that it may lose data upon power-down or a power failure. Thus, the configuration data may also be stored in the repository 102, which is non-volatile memory, for security or reliability, and retrieved from the repository 102 and placed in the memory 124 during operation.

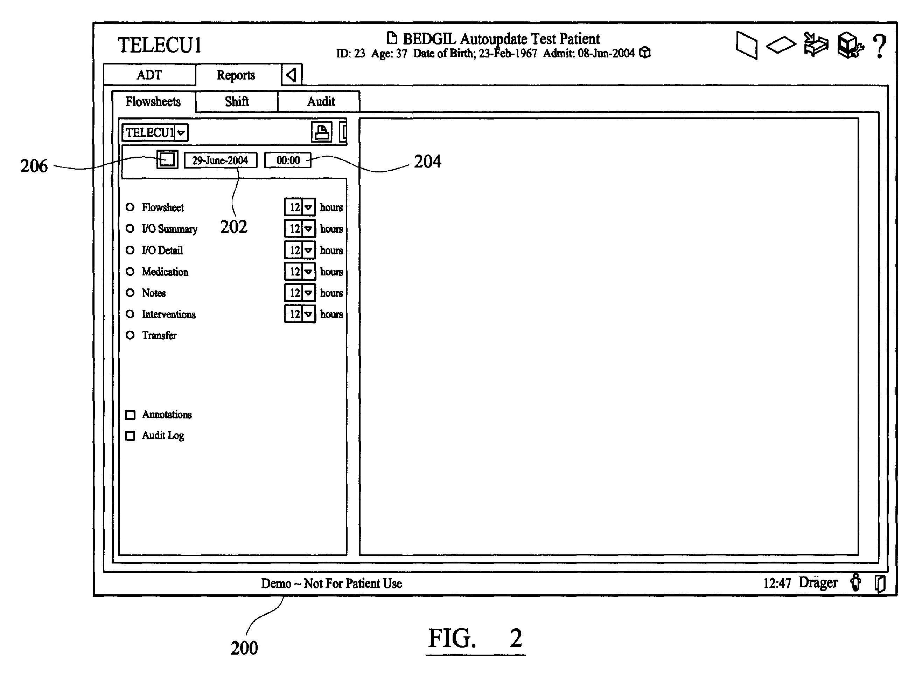

Fig. 2 is an image window of a portion of an exemplary GUI 200, for allowing the user to specify a particular index value. In Fig. 2, a particular date and time, termed an 'Anchor Time', for retrieving physiological parameters may be specified by the user. The user, using the keyboard 114 (Fig. 1), may type a desired date into a text box 202. Alternatively the user may use the mouse 115 to activate a button 206, causing a graphical representation of a calendar (not shown) to be displayed allowing a user to select a date by clicking on the desired day on the calendar. The selected date is then entered automatically in the text box 202. The user may also enter a desired time into the text box 204 using the keyboard 114. In Fig. 2, the specified date and time is 29-June-2004, 00:00 hours. The acquisition processor 104 receives the date and time specified in text boxes 202 and 204 via the user interface processor 122 and stores them in the configuration data 130 in the memory 124. If the data repository 102 stores data for more than one entity, then the acquisition processor 104 stores the specified date and time in the row of the table (Table 4 (above)) associated with the entity, or creates and populates such a row if it does not exist.

Referring again to Fig. 1, before entity related data is retrieved from the data repository 102, the acquisition processor 104 retrieves the anchor time value from the configuration data 130. The acquisition processor 104 then retrieves entity related data from the data repository 102 including data associated with the anchor time value and/or entity related data having index values in the vicinity of the anchor time value. If the retrieved anchor time value is "<<null>>", then the normal default (e.g. the most recent) data is retrieved. If entity related data for more than one entity is stored in the data repository 102, when data for a selected entity is desired, the particular index value for that entity is retrieved from the table (Table 4 (above)) in the configuration data 130 in the memory 124 and that particular index value is used to retrieve data from the data repository 102, as described above.

The acquisition processor 104, conditions the display processor 108 to generate a signal representing an image displaying a report of the retrieved entity related data. The monitor 112 receives this signal and displays the data report image. When a user desires a different type of entity related data, the user selects the desired type using the GUI. The acquisition processor 104 retrieves the desired type of entity related data from the data repository 102 including the data at the anchor time value and/or other entity related data of the desired type in the vicinity of the anchor time value. The acquisition processor 104, conditions the display processor 108 to generate a report image representative signal which is supplied to the monitor 112 which, in turn, displays the entity data report. One skilled in the art understands that multiple display windows may be displayed on the monitor 112, displaying respective reports for different data types at different associated particular index values.

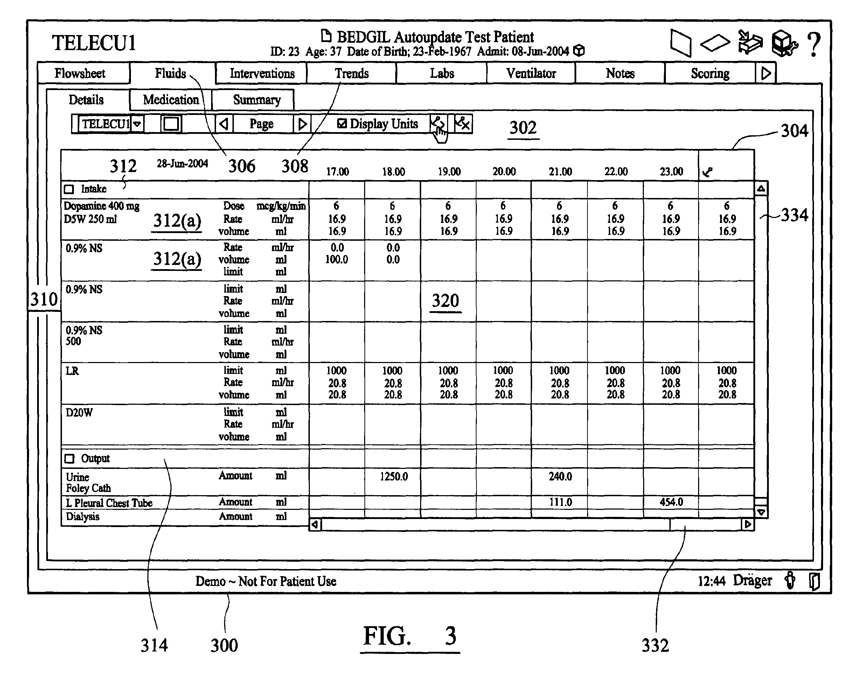

Fig. 3 is an image window of a GUI 300 for displaying a report including entity related data retrieved as described above. In Fig. 3, the physiological parameter of fluid intake and output of a patient is displayed when the user selects the 'Fluids" tab 306, displayed at the top of the GUI 300. The actual data is displayed in the table 320. The rows of table 320 contain data representing a respective physiological parameter. Labels describing the displayed physiological parameter data are displayed at the left hand side 310. For example, fluid intakes 312 include Dopamine 312(a), 0.9% NS 312(b), etc. Fluid outputs 314 are displayed below. The columns of table 320 represent a time index value (date and time) at which the physiological parameters were taken. Labels describing the date and time of the samples are displayed across the top of the table. For example the last column 304 contains data taken at the anchor time, 29-June-2004 at 00:00 hours. Preceding columns contain data taken on 28-June-2004 at hourly intervals from 17:00 to 23:00 hours.

As described above, there may be more data stored in the data repository 102 (Fig. 1) than may be displayed on the GUI image 300 at one time. For example, fluid data for more than the times displayed is typically available. A horizontal scroll bar 332 is available to scroll the display to the right (for more recent times) and to the left (for older times). There are also typically more physiological parameters than may be displayed on the GUI 300 at one time. A vertical scroll bar 334 is available to scroll the display up and down to allow the user to see the available physiological parameters.

In Fig. 3, the particular index value (anchor time) demarcates an end point. The acquisition processor 104 (Fig. 1), therefore, acquires entity related data (fluids data) at index values (times) preceding the particular index value (anchor time). The retrieved entity related data (fluids data) is displayed to the left of the entity related data associated with the specified particular index value (anchor time). One skilled in the art understands that the particular index value may instead demarcate a start point, in which case the acquisition processor 104 acquires entity related data occurring after the particular index value. The retrieved entity related data is displayed to the right of the entity related data associated with the specified particular index value. One skilled in the art also understands that the particular index value may demarcate a center point, in which case the acquisition processor 104 acquires entity related data occurring both before and after the particular index value. The retrieved entity related data is displayed to the right and to the left of the entity related data associated with the specified particular index value. One skilled in the art further understands that the particular index value may demarcate an index value range, in which case the acquisition processor 104 acquires entity related data occurring within the index value range. The retrieved entity related data within the index value range is displayed in the entity related data report.

In the illustrated embodiment, the index value (i.e. time) is displayed running horizontally from left to right, with respective entity related data (i.e. fluid data) running vertically from top to bottom. One skilled in the art understands that the index values may also be displayed running vertically and the entity related values running horizontally.

As described above, other types of entity related data are typically stored in the data repository 102 (Fig. 1). In the illustrated embodiment, the other data types include: 'FlowSheet' data, 'Interventions' data, 'Trends' data, 'Labs' data, 'Ventilator' data, 'Notes' data, 'Scoring' data, and so forth. Image elements, which in Fig. 3 are tabs displayed at the top of the GUI 300, provide access to the other data types. A user may select a different type of entity related (i.e. physiological) data by selecting the desired tab. For example, a user may examine trends data by activating the 'Trends' tab 308. As described above, the acquisition processor 104 retrieves trends data from the data repository 102 and conditions the display processor 108 to generate an image representative signal displaying the trends data on the GUI 300, possibly in a similar format to that illustrated in Fig. 3. The displayed trends data will include trends data taken on "29-June-2004" at 00:00 hours and trends data at times preceding this time.

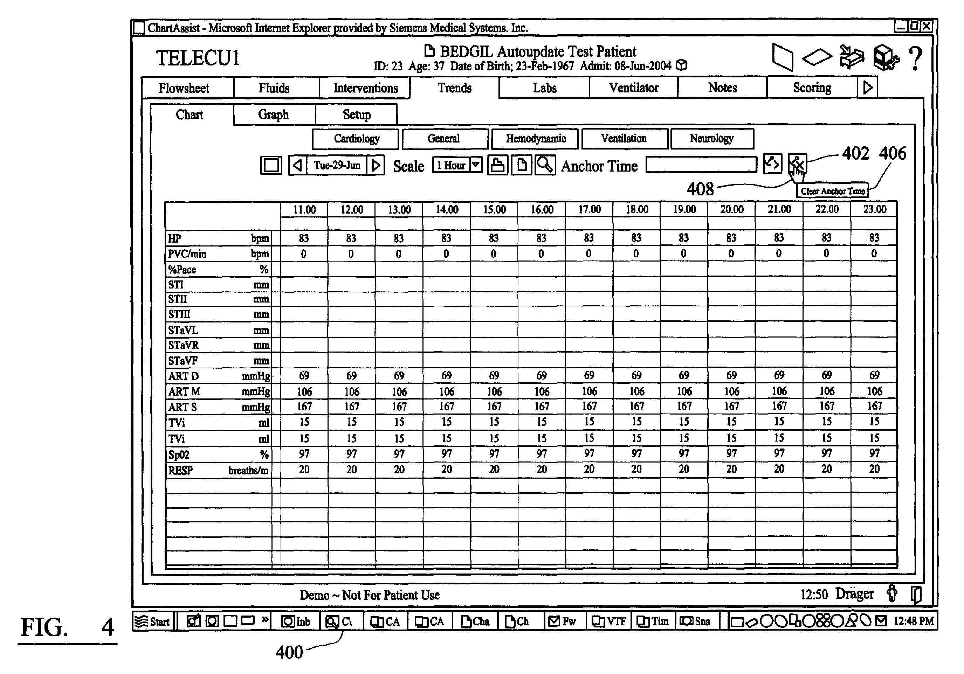

As described above, the acquisition processor 104 may condition the display processor 108 to generate a signal representing a composite image having multiple image windows, similar to that illustrated in Fig. 4. The monitor 112 (Fig. 1) displays the composite image represented by the signal. The multiple image windows may individually display different data types and/or data having different index values (e.g. A composite image including one image window displaying fluids data at 0000 on 28-June-2004, one displaying fluids data at 0800 on 30-June-2004, one displaying trends data at 0800 on 30-June-2004, and so forth). The user may select a desired image window to inspect and interact with using the keyboard 114 and/or mouse 115.

As described above, the user may disable the use of a specified particular index value and allow the data acquisition system to return to using the normal default index value (e.g. most recent times for medical data acquisition systems). Fig. 4 is an image window of a GUI 400 for allowing a user to disable use of a previously specified particular index value (i.e. anchor time). GUI 400 includes a button 402. A cursor, indicated by a hand icon 408 may be controlled by the mouse 115 (Fig. 1). When the cursor is controlled to hover over the button 402 a hint 406 indicates the function of the button 402, i.e. "Clear Anchor Time". When the user activates the button 402, the particular index value (anchor time) is cleared, and the normal default (i.e. most recent) index value is used. The acquisition processor 104 (Fig. 1) receives an indication that the button 402 is activated via the user interface processor 122. In response, the acquisition processor 104 stores a "<<null>>" index value in the configuration data 130, or, in the case where data related to more than one entity is stored in the data repository 102, in the index value column of the row in the table (Table 4 (above)) associated with the current entity. Subsequently, when data is retrieved for the entity, it is retrieved for the normal default index value and for index values around that default value.

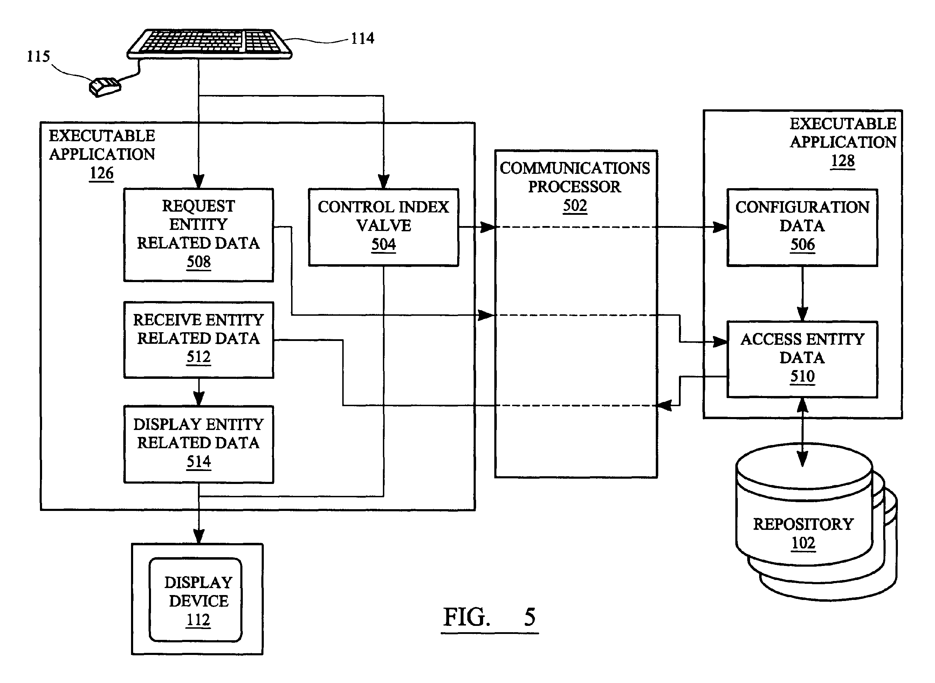

Fig. 5 is a data flow diagram of a portion of executable applications 126 and 128 controlling the operation of the acquisition processor 104 (Fig. 1). A communications processor 502 provides data communications between the executable application 126 and the executable application 128. This may be implemented in any of several ways, such as shared memory, dynamic data exchange (DDE), object linking and embedding (OLE), parameter passing, and so forth. One skilled in the art understands the operation of these and other methods of communicating data from one executable application to another, and how to select an appropriate technique for the illustrated use. In general, the executable application 126 interacts with the user using GUI images (Fig. 2, Fig. 3, Fig. 4) displayed on the display device 112 and input devices, i.e. keyboard 114 and mouse 115, for receiving data and commands from the user. The executable application 128 interacts with the data repository 102 to retrieve entity related data.

An executable procedure 504 in the executable application 126 controls the value of the user specified particular index value. It includes machine readable instructions for conditioning the display device 112 to display the GUI image 200 (Fig. 2). The user interacts with the GUI image 200, to enter data specifying a particular index value using the keyboard 114 and/or mouse 115, as described above. This data is received by the executable procedure 504 which communicates it to an executable procedure 506 in the executable application 128 via the communications processor 502. The executable procedure 506 saves the received data as configuration data. If the data repository 102 contains data related to more than one entity, then the executable procedure 506 stores the received data in an appropriate row of a table (Table 4) containing the configuration data, or creates a row is none exists in the table.

The executable procedure 504 may also condition the display device 112 to display the GUI image 400 (Fig. 4). The user interacts with the GUI 400 using the keyboard 114 and/or mouse 115 to specify that no particular index value is to be used, and the normal default index value is to be used instead. This data is communicated, via the communications processor 502, to the executable procedure 506, which stores the "<<null>>" value in the configuration data, or in the appropriate row of the table (Table 4).

An executable procedure 508 in the executable application 126 receives data from the user input devices 114, 115 indicating that the user is requesting a report of entity related data (Fig. 3) or a specified type of entity related data, e.g. by activating a display element representing a desired report such as tab 306 representing a report 320 of fluids intake 312 and output 314. A request to retrieve entity related data from the data repository 102 is communicated to an executable procedure 510 in the executable application 128 via the communications processor 502. Before retrieving data from the data repository 102, the executable procedure 510 retrieves the currently specified particular index value (i.e. the desired date and time) from the executable procedure 506, or the particular index value for the desired entity (patient) if the data repository stores data for more than one entity. The executable procedure 510 then accesses entity related data of the desired type (fluids data) from the data repository 102 for the particular index value (date and time) and selected entity (patient) and also entity related data for index values around the particular index value, as described above. The retrieved entity related data is communicated via the communications processor 502 from the executable procedure 510 in the executable application 128 to the executable procedure 512 in the executable application 126. This data is supplied to an executable procedure 514, which conditions the display device 112 to display the image 300 of the report of the retrieved entity related data.

One skilled in the art understands that the executable application 126 and the executable application 128 may be generated and supplied by different software suppliers. The communications processor 502 permits these two executable applications 126 and 128 to interoperate.

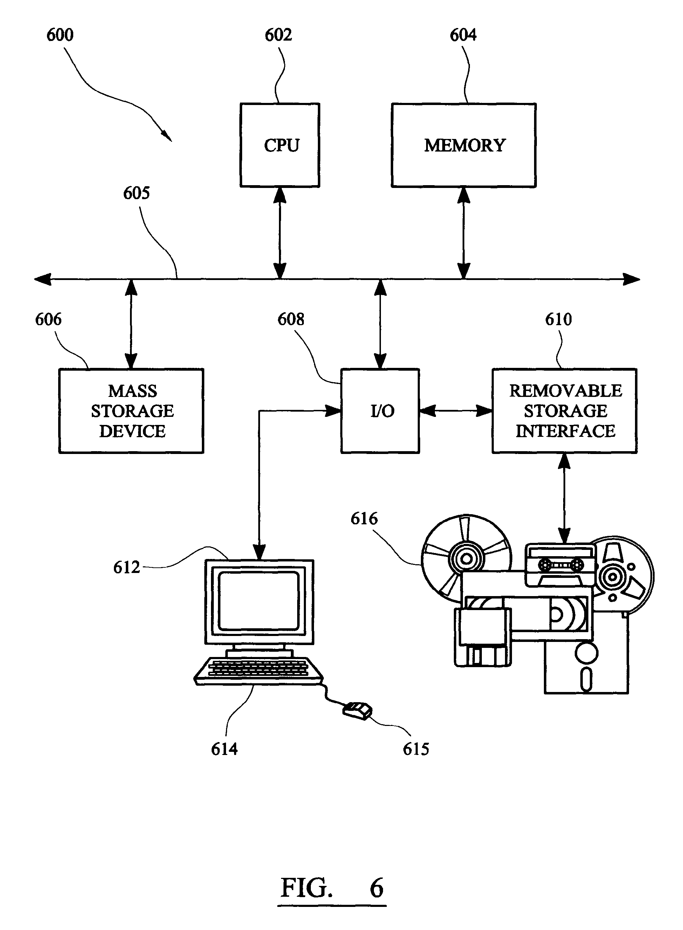

Fig. 6 is a block diagram of a processing system 600 on which the system 100 illustrated in Fig. 1 may be implemented. The processing system 600 includes a central processing unit (CPU) 602, a memory 604, a mass storage device 606, and an input/output interface 608 coupled together by a computer bus 605. The input/output (I/O) interface 608 is coupled to a user interface consisting of a monitor 612, a keyboard 614 and a pointing device, which in the illustrated embodiment is a mouse 615. The I/O interface 608 is also coupled to a removable storage interface 610 capable of retrieving data from or storing data on one or more electronic data storage media 616. The electronic data storage media 616 may include magnetic devices such as reel-to-reel computer tape, cassette tapes, and magnetic disk media such as floppy disks and so forth. The electronic data storage media 616 may also include optical devices, such as digital video disk (DVD) or compact disk (CD) and so forth. One skilled in the art understands that any such electronic data storage media 616 may be used, such as portable storage devices including semiconductor memory integrated circuits. The I/O interface 608 may also be coupled to other peripheral devices (not shown) such as printers or communications devices for communicating with remote systems, local area networks (LANs) or wide area networks (WANs) such as the internet.

In operation, the CPU 602 operates as a processor which executes the machine readable instructions forming executable applications, e.g. 126 and 128, and/or executable procedures, e.g. 504, 506, 508, 510, 512, and 514 (Fig. 5). Those machine readable instructions are stored in the memory 604, which may consist of read-only memory (ROM) and/or read/write memory (RAM). The CPU 602 retrieves the machine readable instructions from the memory 604 and executes them to perform the operations of the data acquisition system, as described above.

In the illustrated embodiment, the I/O processor 608 includes a display processor which, in response to commands from the CPU 602, generates signals representing the GUI display images described above and illustrated in Fig. 2 to Fig. 4, and supplies those image representative signals to the monitor 612 which displays the images. The I/O processor 608 also receives user commands and data from the keyboard 614 and/or mouse 615 and provides that information to the CPU 602. The CPU 602 responds to the received user commands and data to control the operation of the data acquisition system as described above.

Data may be retrieved from and stored in the mass storage device 606. For example, the mass storage device 606 may provide storage for the data repository 102 (Fig. 1). The mass storage device 606 may also store data representing the machine readable instructions forming the executable applications and/or executable procedures. The CPU 602 may retrieve the executable application and/or executable procedures from the mass storage device 606 and store them in the memory 604. The CPU 602 may then retrieve the machine readable instructions from the memory 604 and execute the executable applications and/or executable procedures to perform the data acquisition activities described above.

Data may also be retrieved from and stored in electronic data storage media 616 via the removable storage interface 610. Any data may be stored in and/or retrieved from the electronic data storage media. More specifically, in the illustrated embodiment, the machine readable instructions in the executable applications and/or executable procedures forming the data acquisition system may be stored in an electronic data storage medium. The CPU 602 may condition the I/O processor 608 to retrieve the executable applications and/or executable procedures from the appropriate electronic data storage medium via the removable storage interface 610, and to store the executable application and/or executable procedures in the mass storage device 606 and/or the memory 604. The CPU 602 may then execute the executable application and/or executable procedures in the memory 604 to perform the information acquisition activities described above.

It will be appreciated by persons skilled in the art that the above embodiment has been described by way of example only, and not in any limitative sense, and that various alterations and modifications are possible without departure from the scope of the invention as defined by the appended claims.

## Claims

1. A system for acquiring and providing data relating to an entity to a user, comprising:
at least one repository of entity related data together with information representing an index associated with acquisition of said entity related data;
a source of configuration data identifying a particular value for said index; and
an acquisition processor for accessing said at least one repository to acquire entity related data associated with said particular index value in response to a user command to access entity related data.

2. A system according to claim 1, including one or more of the following features:-
(i) wherein said particular index value demarcates an end point; and
said acquisition processor acquires entity related data preceding said particular index value;
(ii) wherein said particular index value demarcates a start point; and
said acquisition processor acquires entity related data following said particular index value;
(iii) wherein said particular index value demarcates a center point; and
said acquisition processor acquires entity related data preceding and following said particular index value;
(iv) wherein said particular index value comprises an index range; and
said acquisition processor acquires entity related data in said index value range; or
(v) further comprising a display processor for providing data representing a displayed image including said acquired entity related data associated with said particular index value, for presentation to a user.

3. A system according to claim 2, wherein said acquired entity related data is displayed at least one of, (a) to the left, (b) at the center and (c) to the right of said particular index value.

4. A system according to claim 2 or 3, wherein said acquired entity related data is displayed at least one of, (a) to the top, (b) at the center and (c) to the bottom of said particular index value.

5. A system according to any one of the preceding claims, including one or more of the following features:-
(i) wherein said acquisition processor limits the quantity of said acquired entity related data retrieved from said at least one repository in response to a determined available scrollable image size used for displaying said acquired entity related data to a user;
(ii) further comprising:
a user interface processor for receiving a user command to access a particular type of entity related data; and
said acquisition processor accesses said at least one repository to acquire entity related data of said particular type;
(iii) further comprising:
a user interface processor for receiving a user command to access data related to a particular entity related data; and
said acquisition processor accesses said at least one repository to acquire data related to said particular entity; or
(iv) further comprising:
a user interface processor for receiving a user command to access data of a particular type related to a particular entity; and
said acquisition processor accesses said at least one repository to acquire entity related data of said particular type for said particular entity.

6. A system for acquiring and providing particular patient medical record data to a user, comprising:
at least one repository of patient medical record data together with information representing a time associated with acquisition of said medical record data;
a source of configuration data identifying a particular time for a particular patient; and
an acquisition processor for accessing said at least one repository to acquire patient medical record data associated with said particular time in response to user command to access medical record data of said particular patient.

7. A system according to claim 6, including one or more of the following features:
(i) wherein said time associated with acquisition of said medical record data comprises a date;
said particular time for a particular patient comprises a date; and
said acquisition processor acquires patient medical record data associated with a particular date in response to said user command to access medical record data of said particular patient;
(ii) wherein said particular time demarcates an end time; and
said acquisition processor acquires patient medical record data preceding said particular time;
(iii) whereinsaid particular time demarcates a start time; and
said acquisition processor acquires patient medical record data occurring after said particular time;
(iv) wherein said particular time demarcates a center time; and
said acquisition processor acquires patient medical record data preceding and following said particular time;
(v) wherein said particular time comprises a time range; and
said acquisition processor acquires patient medical record data in said time range; or
(vi) further comprising a display processor for providing data representing a displayed image including said acquired patient medical record data associated with said particular time, for presentation to a user.

8. A system according to claim 7, wherein said acquired patient medical record data is displayed at least one of, (a) to the left, (b) at the center and (c) to the right of said particular time.

9. A system according to claim 7 or 8, wherein said acquired patient medical record data is displayed at least one of, (a) to the top, (b) at the center and (c) to the bottom of said particular time.

10. A system according to any one of claims 6 to 9, including one or more of the following features:-
(i) wherein said acquisition processor limits the quantity of said acquired patient medical record data retrieved from said at least one repository in response to a determined available scrollable image size used for displaying said acquired patient medical record data to a user;
(ii) further comprising:
a user interface processor for receiving a user command to access a particular type of patient data of said particular patient; and
said acquisition processor accesses said at least one repository to acquire patient medical record data of said particular type;
(iii) wherein said particular type of patient data is selected from a plurality of different types of patient data associated with a corresponding plurality of images navigable by said user;
(iv) wherein said particular type of patient data is selected from a plurality of different types of patient data associated with a corresponding plurality of portions of a single image, said user command to access said particular type of patient data being received in response to user selection of a displayed image element associated with a corresponding type of patient data in said single image; or
(v) further comprising a communication processor for communicating data identifying said particular time to a second different executable application for use by said second different executable application in initiating acquisition of patient medical record data associated with said particular time in response to a user command to access medical record data of said particular patient received via user selection of an image element in an image display associated with said second different executable application.

11. A system according to claim 10, wherein said plurality of different types of patient data comprise two or more of, (a) blood pressure parameters, (b) ventilation parameters, (c) vital sign parameters, (d) blood oxygen concentration representative parameters and (e) infusion pump parameters associated with fluid delivery.

12. A system supporting navigability and interoperability between different executable applications, comprising:
at least one repository including a plurality of different types of data together with information representing a time associated with data within a particular type, said plurality of different types of data being associated with a plurality of different entities;
first and second different executable applications having access to configuration data identifying a particular time associated with a particular type of data and a particular entity; and
said first and second different executable applications access said at least one repository to acquire a particular type of data associated with said particular time in response to user command to access a particular type of data associated with a particular entity.

13. A system according to claim 12, wherein said first and second different executable applications access said at least one repository to acquire a particular type of data associated with said particular time in response to user commands received via user selection of image elements in image displays associated with said first and second different executable applications, providing presentation of data in a consistent time portion upon navigation between images associated with different executable applications.

14. A method for acquiring and providing entity related data to a user, comprising the activities of:
storing, in at least one repository, entity related data together with information representing an index value associated with acquisition of said entity related data;
receiving user entered configuration data identifying a particular index value; and
accessing said at least one repository to acquire entity related data associated with said particular index value in response to user command to access entity related data.

15. A method for acquiring and providing particular patient medical record data to a user, comprising the activities of:
storing, in at least one repository, patient medical record data together with information representing a time associated with acquisition of said medical record data;
receiving user entered configuration data identifying a particular time for a particular patient; and
accessing said at least one repository to acquire patient medical record data associated with said particular time in response to user command to access medical record data of said particular patient.

16. An electronic data storage medium incorporating machine readable instructions for performing the activities of claim 14 or 15.
